# EUROPEAN PATENT APPLICATION

(11) **EP 1 702 590 A2**
(43) Date of publication of application: **20.09.2006**
(21) Application number: 06003706.6
(22) Date of filing: 23.02.2006
(51) Int. Cl.: A61F 2/38

(54) **Modular tibial implant with a mortise coupling**

(30) Priority: 26.02.2005 US 67184
(71) Applicant: Zimmer Technology, Inc., Chicago, Illinois 60606 (US)
(72) Inventor: Pendleton, John Edward, Ft. Wayne, IN 46804 (US); Dykema, Scott E., Warsaw, IN 46582 (US)
(74) Representative: Manitz, Finsterwald & Partner GbR

(57) **Abstract**

A modular tibial implant apparatus (100) includes a tibial plate (120) and a tibial stem (140) mortised to the tibial plate (120). A method for anchoring a tibial plate and a tibial stem relative to a proximal tibia includes anchoring the tibial stem in the proximal tibia and mortising the tibial plate to the tibial stem.

## Description

### FIELD OF THE INVENTION

The present invention relates generally to the field of orthopaedics, and, more particularly, to a modular tibial implant with a mortise coupling.

### BACKGROUND

Total joint arthroplasty ("joint replacement") is the surgical replacement of a joint with a prosthesis. A typical knee prosthesis has three main components: a femoral implant, a tibial implant, and a tibio-femoral insert. In general, the femoral implant is designed to replace the distal femoral condyles. The femoral implant is typically made from metal. It typically includes medial and lateral rounded surfaces for emulating the medial and lateral condyles, respectively, with a middle section therebetween for emulating the patella sulcus/trochlear region of the distal femur. In general, the tibial implant is designed to support and align the tibio-femoral insert. The tibial implant is also typically made from metal. It typically includes a substantially planar tray or plate portion ("tibial plate") for supporting the insert, and an elongated anchor ("tibial stem") extending away from the tibial plate for anchoring the tibial implant in the intramedullary canal of the proximal tibia. In general, the tibio-femoral insert is designed to replace the tibial plateau and the meniscus of the knee. It is typically somewhat disk-shaped, and typically includes one or more substantially planar surfaces for bearing on the tibial plate and one or more generally concave surfaces for bearing against the femoral implant. The insert is typically made of a strong, smooth, low-wearing plastic.

In a traditional knee replacement, the surgeon makes a rather lengthy anterior incision spanning the distal femur, the knee, and the proximal tibia; separates the distal femur and proximal tibia from the surrounding tissues; hyperflexes, distally extends, and/or otherwise distracts the proximal tibia from the distal femur to make room for specialized guides and saws; and uses the guides and saws to prepare these bones for receiving the prosthetics. The surgeon may apply cement to the distal femur and/or to the proximal tibia to help hold the femoral implant and/or tibial implant, respectively, in place. Alternatively, cementless implants may be used. Further, the surgeon drives the femoral implant onto the cut surface of the distal femur and drives the tibial stem generally longitudinally into the intramedullary canal of the proximal tibia. Finally, the surgeon attaches the tibio-femoral insert to the tibial plate and closes the surgical site.

In contrast to a traditional knee replacement, knee replacement through minimally invasive surgery employs, among other things, smaller incisions, which tend to reduce tissue traumas and accelerate post-operative recoveries. However, because minimally invasive surgery reduces the size of the surgical site, it also generally reduces the amount of space available for inserting, aligning, and securing unitary tibial implants with long, inseparable tibial stems.

### SUMMARY OF THE INVENTION

The present invention provides a modular tibial implant apparatus including a tibial plate and a tibial stem mortised to the tibial plate.

The present invention provides an apparatus for holding a tibio-femoral insert relative to a proximal tibia. The apparatus includes a means for supporting the tibio-femoral insert, a means for anchoring the supporting means relative to the proximal tibia and a means, interposed between the supporting means and the anchoring means, for mortising the anchoring means to the supporting means.

The present invention provides a method for anchoring a tibial plate and a tibial stem relative to a proximal tibia. The method includes anchoring the tibial stem in the proximal tibia and mortising the tibial plate to the tibial stem.

The above-noted features and advantages of the present invention, as well as additional features and advantages, will be readily apparent to those skilled in the art upon reference to the following detailed description and the accompanying drawings, which include a disclosure of the best mode of making and using the invention presently contemplated.

### BRIEF DESCRIPTION OF THE DRAWINGS

FIG. 1 shows a perspective view of an exemplary modular tibial implant apparatus according to the present invention;

FIG. 2 shows an exploded perspective view of the exemplary apparatus of FIG. 1;

FIG. 3 shows an anterior plan view of the exemplary tibial stem of the apparatus of FIG. 1;

FIG. 4 shows a superior plan view of the exemplary tibial stem of the apparatus of FIG. 1;

FIG. 5 shows a posterior plan view of the exemplary tibial plate of the apparatus of FIG. 1;

FIG. 6 shows an inferior plan view of the exemplary tibial plate of the apparatus of FIG. 1;

FIG. 7 shows a perspective view of an exemplary alternative modular tibial implant apparatus according to the present invention;

FIG. 8 shows an exploded perspective view of the exemplary apparatus of FIG. 7;

FIG. 9 shows an anterior plan view of the exemplary tibial stem of the apparatus of FIG. 7;

FIG. 10 shows a superior plan view of the exemplary tibial stem of the apparatus of FIG. 7;

FIG. 11 shows a posterior plan view of the exemplary tibial plate of the apparatus of FIG. 7; and

FIG. 12 shows an inferior plan view of the exemplary tibial plate of the apparatus of FIG. 7.

### DETAILED DESCRIPTION OF EXEMPLARY EMBODIMENT(S)

Like reference numerals refer to like parts throughout the following description and the accompanying drawings. As used herein, the terms "medial," "medially," and the like mean pertaining to the middle, in or toward the middle, and/or nearer to the middle of the body when standing upright. Conversely, the terms "lateral," "laterally," and the like are used herein as opposed to medial. For example, the medial side of the knee is the side closest to the other knee and the closest sides of the knees are medially facing, whereas the lateral side of the knee is the outside of the knee and is laterally facing. Further, as used herein the term "superior" means closer to the top of the head and/or farther from the bottom of the feet when standing upright. Conversely, the term "inferior" is used herein as opposed to superior. For example, the heart is superior to the stomach and the superior surface of the tongue rests against the palate, whereas the stomach is inferior to the heart and the palate faces inferiorly toward the tongue. Additionally, as used herein the terms "anterior," "anteriorly," and the like mean nearer the front or facing away from the front of the body when standing upright, as opposed to "posterior," "posteriorly," and the like, which mean nearer the back or facing away from the back of the body. Additionally, as used herein, the term "tenon" and inflections thereof are intended in the broad conventional sense to indicate a projecting part for insertion into a corresponding hole or notch ("mortise") to form a joint, and, accordingly, the term "mortise" and inflections thereof are intended in the broad conventional sense to indicate a hole or notch in one member or part configured to fit a corresponding projection ("tenon") extending from another member or part to join the two parts together and, where applicable, to indicate joining two parts together via mortise-tenon joint. Also, as used herein the terminology "taper couple" and inflections thereof mean to fasten together via a taper joint. In general, a taper joint or taper coupling is formed by pressing together ("press-fitting") a male part ("male taper") and a female part ("female taper") having impinging angled or flared surfaces.

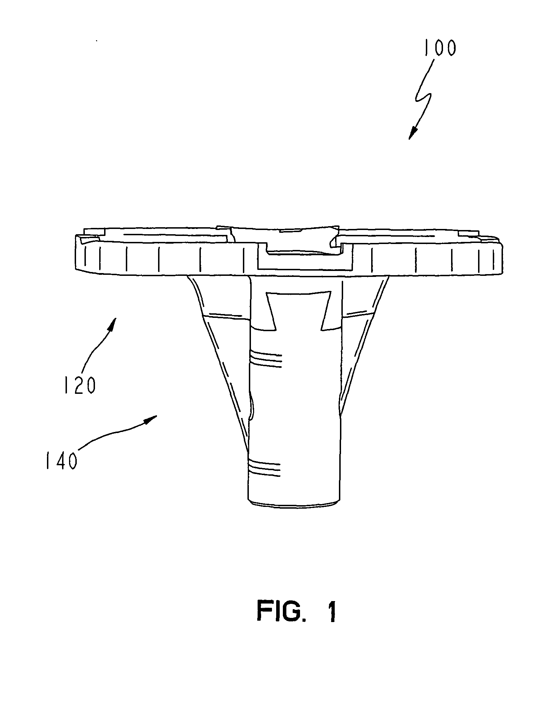

FIG. 1 shows a perspective view of an exemplary modular tibial implant apparatus 100 according to the present invention. Apparatus 100 includes an exemplary tibial plate 120 and an exemplary tibial stem 140. Among other things, plate 120 is configured to support and align a conventional tibio-femoral insert (not shown) in a known manner and plate 120 is further configured to connect to stem 140 in accordance with the exemplary embodiment of the present invention as discussed further below. In the exemplary embodiment, plate 120 is made from a titanium alloy. In alternative embodiments, plate 120 may be made from a cobalt chrome alloy or any other suitable biocompatible material(s).

Among other things, stem 140 is configured to anchor into a typical proximal tibia (not shown) in a known manner and stem 140 is further configured to connect to plate 120 in accordance with the exemplary embodiment of the present invention as discussed further below. In the exemplary embodiment, stem 140 is made from a titanium alloy. In alternative embodiments, stem 140 may be made from a cobalt chrome alloy or any other suitable biocompatible material(s).

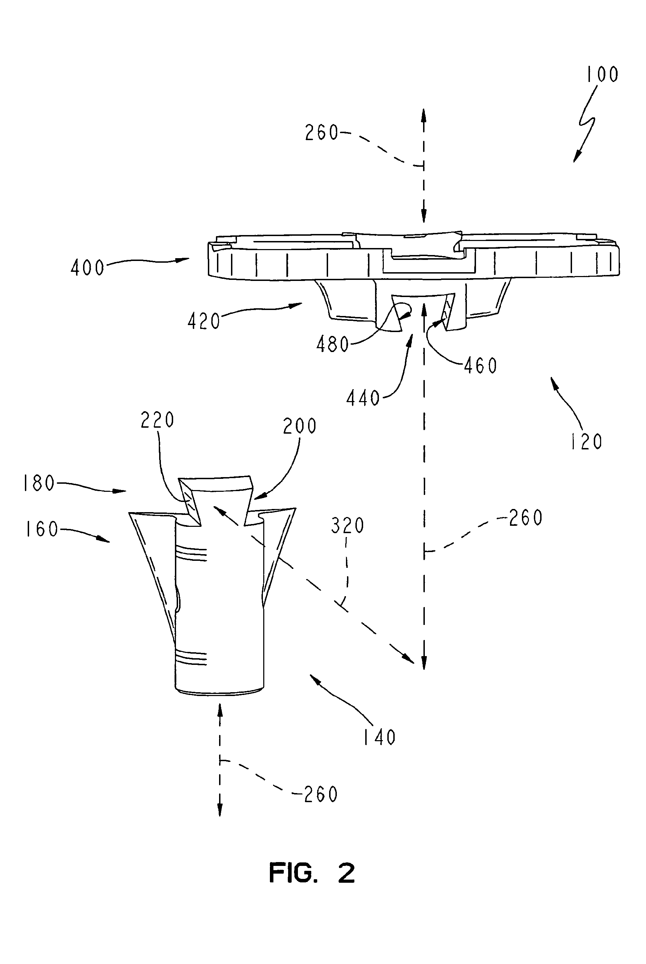

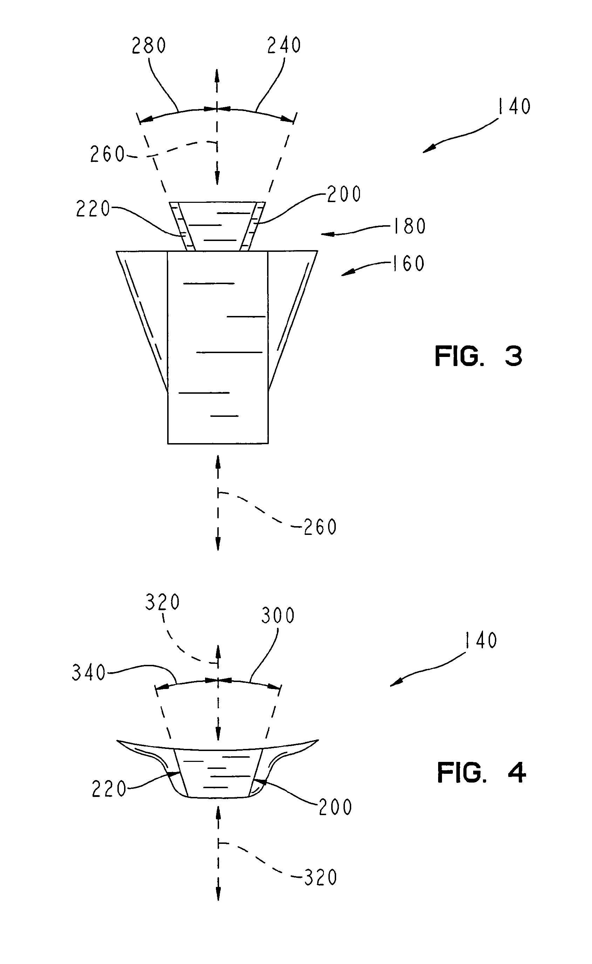

FIG. 2 shows an exploded perspective view of apparatus 100. As discernable in FIG. 2, stem 140 includes an anchor portion 160. Among other things, portion 160 is configured as known to anchor into the typical proximal tibia. Stem 140 also includes a tenon 180 extending generally superiorly from portion 160. Among other things, tenon 180 is configured to cooperate with plate 120 to concurrently mortise and taper couple stem 140 to plate 120 in accordance with the exemplary embodiment of the present invention. Tenon 180 includes a medial planar surface 200 and a lateral planar surface 220. Surface 200 extends generally superiorly and somewhat medially from portion 160 at a dovetail angle 240 (see FIG. 3) of about 30 degrees relative to an inferiorly-superiorly extending medial-lateral split-line 260 while surface 220 extends generally superiorly and somewhat laterally from portion 160 at a dovetail angle 280 (see FIG. 3) of about 30 degrees relative to split-line 260 so as to dovetail tenon 180 generally medially-laterally outward as tenon 180 extends from portion 160. Additionally, surface 200 traverses generally posteriorly-anteriorly and somewhat medially-laterally over portion 160 at a taper angle 300 (see FIG. 4) of about 30 degrees relative to a posteriorly-anteriorly extending medial-lateral split-line 320 while surface 220 traverses generally posteriorly-anteriorly and somewhat laterally-medially over portion 160 at a taper angle 340 (see FIG. 4) of about 30 degrees relative to split-line 320 so as to taper tenon 180 generally medially-laterally inward as tenon 180 posteriorly-anteriorly traverses portion 160. It should be appreciated that split-line 260 is perpendicular to split-line 320.

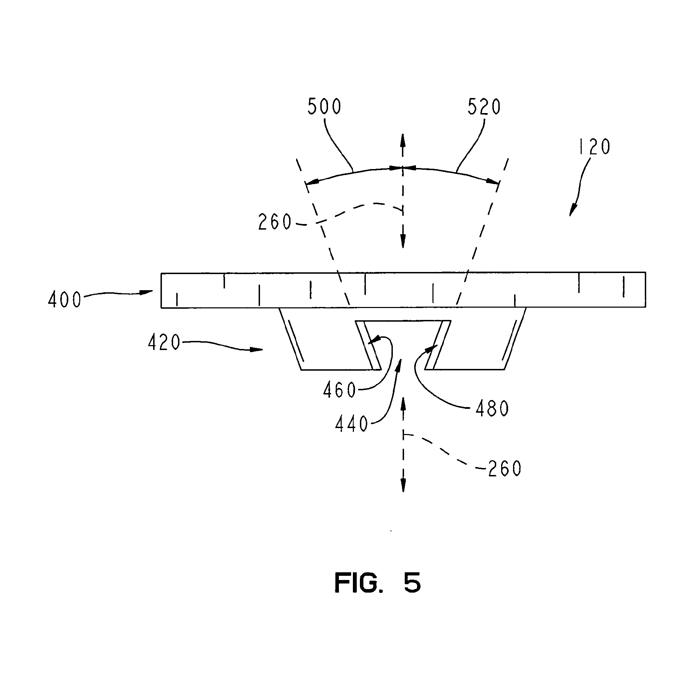

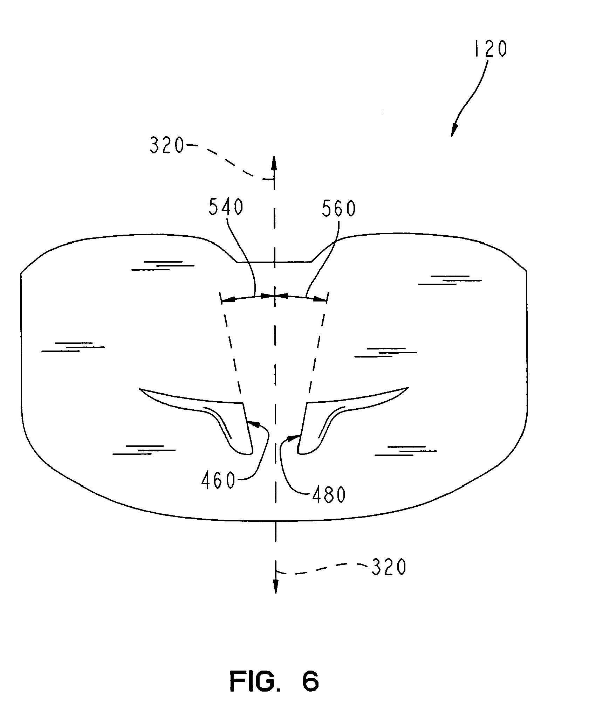

As further discernable in FIG. 2, plate 120 includes a tray portion 400. Among other things, portion 400 is configured as known to support and align the conventional tibio-femoral insert. Plate 120 also includes a base 420 extending generally posteriorly from portion 400. Among other things, base 420 is configured to cooperate with stem 140 to concurrently mortise and taper couple plate 120 to stem 140 in accordance with the exemplary embodiment of the present invention. Accordingly, base 420 defines a mortise 440. Among other things, mortise 440 is configured press or interference fit securely around tenon 180 such that, when tenon 180 is inserted into mortise 440, tenon 180 and mortise 440 dovetail together in opposition to separation along split-line 260 and tenon 180 and mortise 440 further concurrently taper couple together in opposition to separation along split-line 320. Mortise 440 is partly defined by a medial planar surface 460 and a lateral planar surface 480. Surface 460 extends generally superiorly and somewhat medially into base 420 at a dovetail angle 500 (see FIG. 5) of about 30 degrees relative to split-line 260 while surface 480 extends generally superiorly and somewhat laterally into base 420 at a dovetail angle 520 (see FIG. 5) of about 30 degrees relative to split-line 260 so as to dovetail mortise 440 generally medially-laterally outward as mortise 440 extends into base 420. Additionally, surface 460 extends generally posteriorly-anteriorly and somewhat medially-laterally through base 420 at a suitable taper angle 540 (see FIG. 6) relative to split-line 320 while surface 480 extends generally posteriorly-anteriorly and somewhat laterally-medially through base 420 at a suitable taper angle 560 (see FIG. 6) relative to split-line 320 so as to suitably taper mortise 440 generally medially-laterally inward as mortise 440 posteriorly-anteriorly extends through base 420. In the exemplary embodiment, angle 540 is equal to or somewhat less than angle 300 (but not less than about 17 degrees less than angle 300), and angle 560 is equal to or somewhat less than angle 340 (but not less than about 17 degrees less than angle 340) to facilitate a suitably secure yet suitably easy to engage taper coupling between tenon 180 and mortise 440.

FIG. 3 shows an anterior plan view of stem 140. Portion 160, tenon 180 (including surface 200 and surface 220), angle 240, split-line 260, and angle 280, among other things, are all at least partially discernable in FIG. 3.

FIG. 4 shows a superior plan view of stem 140. Surface 200, surface 220, angle 300, split-line 320, and angle 340, among other things, are all at least partially discernable in FIG. 4.

FIG. 5 shows a posterior plan view of plate 120. Split-line 260, portion 400, base 420 (including mortise 440, surface 460, and surface 480), angle 500, and angle 520, among other things, are all at least partially discernable in FIG. 5.

FIG. 6 shows an inferior plan view of plate 120. Split-line 320, surface 460, surface 480, angle 540, and angle 560, among other things, are all at least partially discernable in FIG. 6.

To use apparatus 100, portion 160 of stem 140 is anchored into the proximal tibia (before plate 120 is connected to stem 140) via conventional broaching and impacting techniques, which may or may not include an application of bone cement around portion 160, as desired. It should be appreciated that installing stem 140 separately from plate 120 requires less clearance space than that which would be required for installing them together as a unitary part. After portion 160 of stem 140 is anchored into the proximal tibia, plate 120 is positioned generally anteriorly to tenon 180 such that mortise 440 is aligned along split-line 320. Plate 120 is then moved posteriorly to slide mortise 440 around tenon 180 (and, thus, insert tenon 180 into mortise 440), which mortises (and, more specifically, dovetails) tenon 180 into mortise 440 in opposition to separation along split-line 260 and concurrently taper couples tenon 180 into mortise 440 in opposition to separation along split-line 320. After plate 120 is connected to stem 140 in the foregoing manner, plate 120 may suitably support and align the conventional tibio-femoral insert.

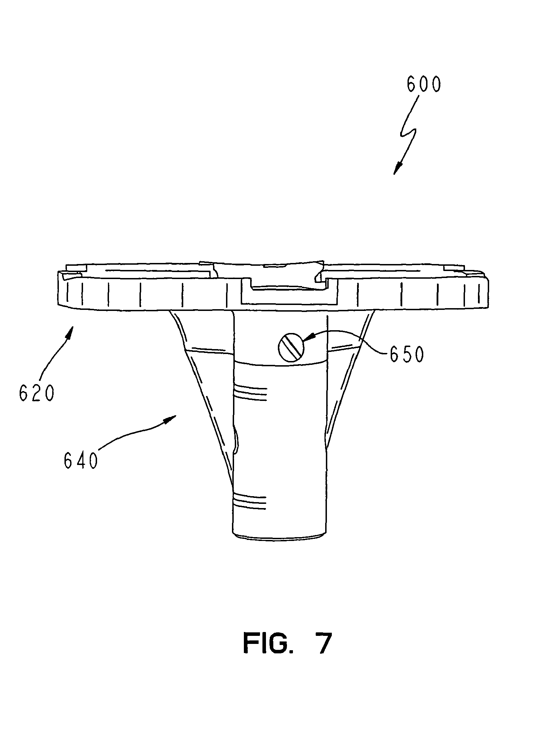

FIG. 7 shows a perspective view of an exemplary alternative modular tibial implant apparatus 600 according to the present invention. Apparatus 600 includes an exemplary tibial plate 620 and an exemplary tibial stem 640. Among other things, plate 620 is configured to support and align a conventional tibio-femoral insert (not shown) in a known manner and plate 620 is further configured to connect to stem 640 in accordance with the exemplary embodiment of the present invention as discussed further below. In the exemplary embodiment, plate 620 is made from a titanium alloy. In alternative embodiments, plate 620 may be made from a cobalt chrome alloy or any other suitable biocompatible material(s).

Among other things, stem 640 is configured to anchor into a typical proximal tibia (not shown) in a known manner and stem 640 is further configured to connect to plate 620 in accordance with the exemplary embodiment of the present invention as discussed further below. In the exemplary embodiment, stem 640 is made from a titanium alloy. In alternative embodiments, stem 640 may be made from a cobalt chrome alloy or any other suitable biocompatible material(s).

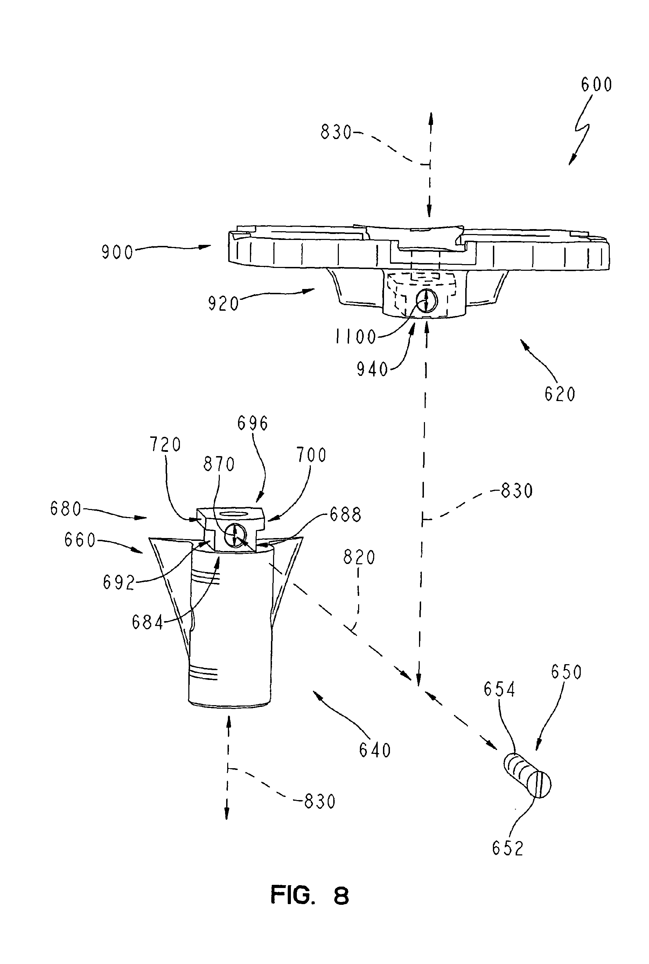

Apparatus 600 further includes a bolt 650. Bolt 650 includes a head 652 (see FIG. 8), and further includes a screw-threaded shaft 654 extending through plate 620 into stem 640 (see FIG. 8). Among other things, bolt 650 is configured to help maintain the connection of plate 620 to stem 640. In the exemplary embodiment, bolt 650 is made from a titanium alloy. In alternative embodiments, bolt 650 may be made from a cobalt chrome alloy or any other suitable biocompatible material(s).

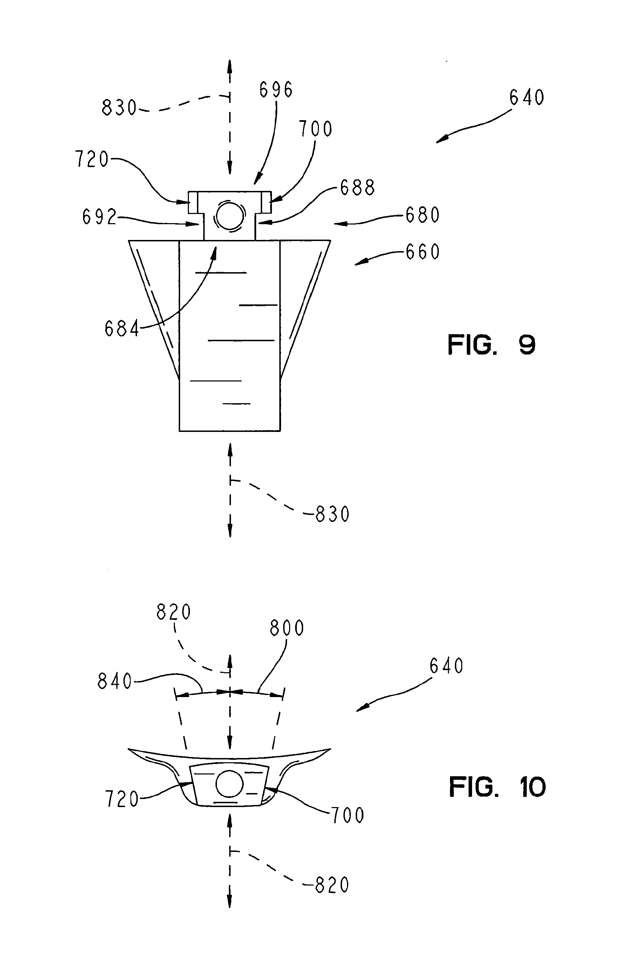

FIG. 8 shows an exploded perspective view of apparatus 600. As discernable in FIG. 8, stem 640 includes an anchor portion 660. Among other things, portion 660 is configured as known to anchor into the typical proximal tibia. Stem 640 also includes a generally T-shaped tenon 680 extending generally superiorly from portion 660. Among other things, tenon 680 is configured to cooperate with plate 620 to concurrently mortise and taper couple stem 640 to plate 620 in accordance with the exemplary embodiment of the present invention. Tenon 680 includes a neck portion 684 extending generally superiorly from portion 660. Portion 684 includes a medial planar surface 688 extending generally superiorly from and generally posteriorly-anteriorly over portion 660, and further includes a lateral planar surface 692 extending generally superiorly from and generally posteriorly-anteriorly over portion 660. Tenon 680 also includes a head portion 696 extending generally superiorly from portion 684. Portion 696 includes a medial planar surface 700 and a lateral planar surface 720. Surface 700 extends generally superiorly and is somewhat medially disposed relative to surface 688, while surface 720 extends generally superiorly and is somewhat laterally disposed relative to surface 692. Additionally, surface 700 extends generally posteriorly-anteriorly and somewhat medially-laterally at a taper angle 800 (see FIG. 10) of about 30 degrees relative to a posteriorly-anteriorly extending medial-lateral split-line 820 while surface 720 extends generally posteriorly-anteriorly and somewhat laterally-medially at a taper angle 840 (see FIG. 10) of about 30 degrees relative to split-line 820 so as to taper portion 696 generally medially-laterally inward as portion 696 posteriorly-anteriorly traverses portion 684. It should be appreciated that split-line 820 is perpendicular to an inferiorly-superiorly extending medial-lateral split-line 830. Tenon 680 also defines an anteriorly opening generally cylindrical screw-threaded channel 870.

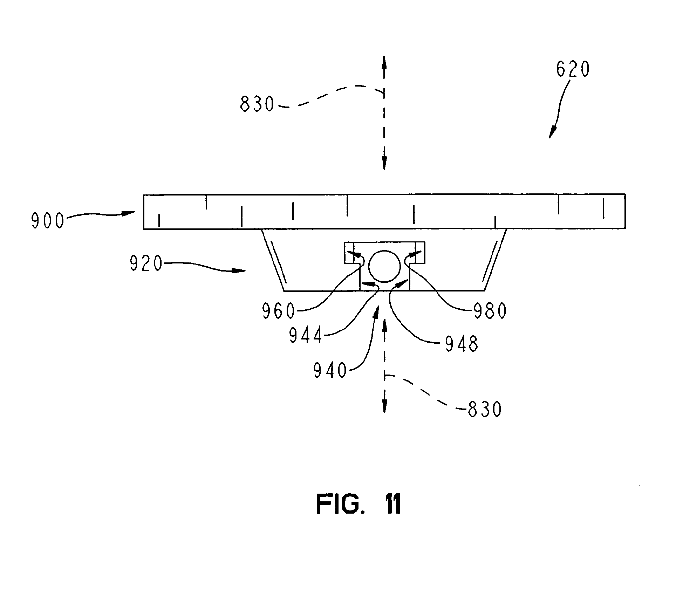

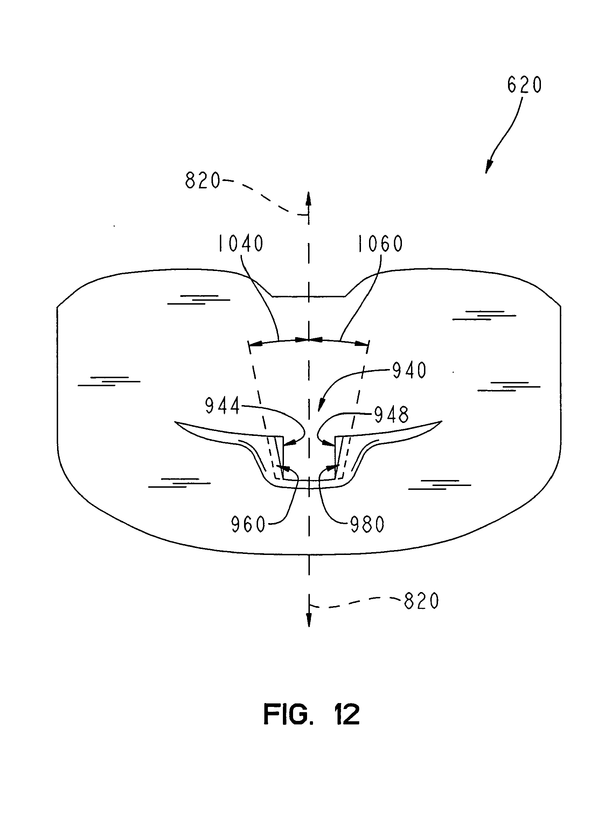

As further discernable in FIG. 8, plate 620 includes a tray portion 900. Among other things, portion 900 is configured as known to support and align the conventional tibio-femoral insert. Plate 620 also includes a base 920 extending generally posteriorly from portion 900. Among other things, base 920 is configured to cooperate with stem 640 to concurrently mortise and taper couple plate 620 to stem 640 in accordance with the exemplary embodiment of the present invention. Accordingly, base 920 defines a generally T-shaped mortise 940 (see also FIG. 11 and FIG. 12). Among other things, mortise 940 is configured press or interference fit securely around tenon 680 such that, when tenon 680 is inserted into mortise 940, tenon 680 and mortise 940 mortise together in opposition to separation along split-line 830 and tenon 680 and mortise 940 further concurrently taper couple together in opposition to separation along split-line 820. Mortise 940 is defined in part by a distal medial planar surface 944 (see FIG. 11 and FIG. 12) extending generally superiorly into and generally posteriorly-anteriorly into base 920, and defined in part by a distal lateral planar surface 948 extending generally superiorly into and generally posteriorly-anteriorly into base 920. Mortise 940 is also defined in part by a proximal medial planar surface 960 and a proximal lateral planar surface 980 (see FIG. 11 and FIG. 12). Surface 960 is somewhat medially disposed from surface 944 and extends generally posteriorly-anteriorly and somewhat medially-laterally into base 920 at a suitable taper angle 1040 (see FIG. 12) relative to split-line 820 while surface 980 is somewhat laterally disposed from surface 948 and extends generally posteriorly-anteriorly and somewhat laterally-medially into base 920 at a suitable taper angle 1060 (see FIG. 12) relative to split-line 820. In the exemplary embodiment, angle 1040 is equal to or somewhat less than angle 800 (but not less than about 17 degrees less than angle 800), and angle 1060 is equal to or somewhat less than angle 840 (but not less than about 17 degrees less than angle 840) to facilitate a suitably secure yet suitably easy to engage taper coupling between tenon 680 and mortise 940. Additionally, base 920 defines an anteriorly positioned aperture 1100. Bolt 650 (including head 652 and shaft 654), among other things, is also at least partially discernable in FIG. 8.

FIG. 9 shows an anterior plan view of stem 640. Portion 660, tenon 680 (including portion 684, surface 688, surface 692, portion 696, surface 700, and surface 720), and split-line 830, among other things, are all at least partially discernable in FIG. 9.

FIG. 10 shows a superior plan view of stem 640. Surface 700, surface 720, angle 800, split-line 820, and angle 840, among other things, are all at least partially discernable in FIG. 10.

FIG. 11 shows a posterior plan view of plate 620. Split-line 830, portion 900, and base 920 (including mortise 940, surface 944, surface 948, surface 960, and surface 980), among other things, are all at least partially discernable in FIG. 11.

FIG. 12 shows an inferior plan view of plate 620. Split-line 820, surface 944, surface 948, surface 960, surface 980, angle 1040, and angle 1060, among other things, are all at least partially discernable in FIG. 12.

To use apparatus 600, portion 660 of stem 640 is anchored into the proximal tibia (before plate 620 is connected to stem 640) via conventional broaching and impacting techniques, which may or may not include an application of bone cement around portion 660, as desired. It should be appreciated that installing stem 640 separately from plate 620 requires less clearance space than that which would be required for installing them together as a unitary part. After portion 660 of stem 640 is anchored into the proximal tibia, plate 620 is positioned generally anteriorly to tenon 680 such that mortise 940 is aligned along split-line 820. Plate 620 is then moved posteriorly to slide mortise 940 around tenon 680 (and, thus, insert tenon 680 into mortise 940), which mortises tenon 680 into mortise 940 in opposition to separation along split-line 830 and concurrently taper couples tenon 680 into mortise 940 in opposition to separation along split-line 820. Next, shaft 652 of bolt 650 is inserted through aperture 1100 into channel 870 and bolt 650 is screw tightened to help maintain the connection. After plate 620 is connected to stem 640 in the foregoing manner, plate 620 may suitably support and align the tibio-femoral insert.

The apparatus of the invention can be used in a method for anchoring a tibial plate and a tibial stem relative to a proximal tibia, the method comprising: anchoring the tibial stem in the proximal tibia; and mortising the tibial plate to the tibial stem. Preferably, the method further includes taper coupling the tibial stem to the tibial plate concurrently with the mortising. Preferably, the mortising includes dovetailing the tibial stem to the tibial plate. Preferably, the mortising includes dovetailing the tibial stem to the tibial plate.

The foregoing description of the invention is illustrative only, and is not intended to limit the scope of the invention to the precise terms set forth. Further, although the invention has been described in detail with reference to certain illustrative embodiments, variations and modifications exist within the scope and spirit of the invention as described and defined in the following claims.

## Claims

1. Apparatus for holding a tibio-femoral insert relative to a proximal tibia, the apparatus comprising:
first means (120; 620) for supporting the tibio-femoral insert;
second means (140; 640) for anchoring the supporting means (120; 620) relative to the proximal tibia; and
third means (180, 440; 680, 940, 650), interposed between the first means (120; 620) and the second means (140; 640), for mortising the anchoring means (140; 640) to the supporting means (120; 620).

2. Apparatus according to claim 1,
**characterized in that**
the apparatus further comprises a fourth means (200, 220, 460, 480; 700, 720, 960, 980), integrated with the third means, for taper coupling the second means to the first means.

3. Apparatus according to claim 1 or 2,
**characterized in that**
the third means includes a means (200, 220) for dovetailing the anchoring means (140) to the supporting means (120).

4. Apparatus according to any of the preceding claims,
**characterized in that**
the apparatus is a modular tibial implant apparatus comprising:
a tibial plate (120; 620); and
a tibial stem (140; 640) mortised to the tibial plate (120; 620).

5. Apparatus according to claim 4,
**characterized in that**
the tibial plate defines a mortise (440; 940) and the tibial stem includes a tenon (180; 680) positioned in the mortise (440; 940).

6. Apparatus according to claim 5,
**characterized in that**
the tenon (180; 680) taper couples into the mortise (440; 940).

7. Apparatus according to claim 5,
**characterized in that**
the tenon (180) dovetails into the mortise (440).

8. Apparatus according to claim 7,
**characterized in that**
the tenon (180) also taper couples into the mortise (440).
